# EUROPEAN PATENT APPLICATION

(11) **EP 3 120 924 A1**
(43) Date of publication of application: **25.01.2017**
(21) Application number: 15178316.4
(22) Date of filing: 24.07.2015
(51) Int. Cl.: B01J 19/00, B01D 53/14, C07C 68/00, C08G 64/00

(54) **APPARATUS AND PROCESS FOR RELIEVING PRESSURE IN A CHEMICAL PLANT**

(71) Applicant: Shell Internationale Research Maatschappij B.V., 2596 HR Den Haag (NL)
(72) Inventor: VROUWENVELDER, Cornelis Leonardus Maria, 1031 HW Amsterdam (NL); VAPORCIYAN, Garo Garbis, Houston, TX 77079 (US)
(74) Representative: Matthezing, Robert Maarten

(57) **Abstract**

An apparatus and a process for relieving pressure in a chemical plant are disclosed. A gas stream is supplied to a scrubber comprising an organic solvent.

## Description

### Field of the invention

The present invention relates to an apparatus and a process for relieving pressure in a chemical plant.

### Background of the invention

In a chemical plant there are a variety of circumstances that can lead to an undesirable and potentially dangerous increase in pressure in vessels such as storage tanks, distillation columns, accumulator vessels or reactors. The pressure can increase because of an emergency situation such as a fire, a runaway reaction, overheating or overfilling with too much liquid. Vessels typically have a relief valve that can be opened when the pressure is too high. The relief valve leads to a relief line, and gases can be released from the vessel via the relief line. Often the relief line leads to a flare where the gas stream is combusted. Sometimes the relief line may simply be vented, although this is clearly not preferred as the gas stream will then enter the atmosphere. The relief line is often directed to a scrubber wherein certain gaseous components may be removed before the gas stream is supplied to the flare or is vented.

Problems can arise in chemical plants that use or produce components that have low melting points e.g. phenol which solidifies at 40-43°C, diphenyl carbonate which solidifies at 78-81°C or ethyl carbonate which solidifies at 40-44°C. There is a risk that the temperature will fall below these melting points in relief, flare or vent lines and this can lead to solidification of components, which can cause plugging or blockage. Lines may be designed to mitigate this risk; short lines can help as there is less opportunity for the gases to cool in the line, and heated lines may also be beneficial. The present inventors have sought to provide improved apparatus for relieving pressure in a chemical plant, wherein plugging or blockage of relief, flare or vent lines is avoided.

### Summary of the invention

Accordingly, the present invention provides an apparatus for relieving pressure in a chemical plant, comprising:
a vessel that has a relief valve leading to a relief line, wherein the relief valve opens when the pressure in the vessel goes above a threshold value thereby allowing a gas stream from the vessel to enter the relief line; and
a scrubber, wherein the relief line provides the gas stream from the vessel to the scrubber, wherein a solvent is present in the scrubber and the solvent contacts the gas stream in the scrubber and wherein the solvent is an organic solvent.

The present invention further provides a process for relieving pressure in a chemical plant, comprising steps of:
opening a relief valve between a vessel and a relief line when the pressure in the vessel goes above a threshold value, thereby allowing a gas stream to enter the relief line;
supplying the gas stream from the relief line to a scrubber;
contacting the gas stream with a solvent in the scrubber; wherein the solvent is an organic solvent.

The present inventors have found that by using an organic solvent in the apparatus and process of the invention it is possible to remove the components having a low melting point in the scrubber, thereby reducing the risk of solidification of components in the relief, vent and flare lines.

### Description of the Figures

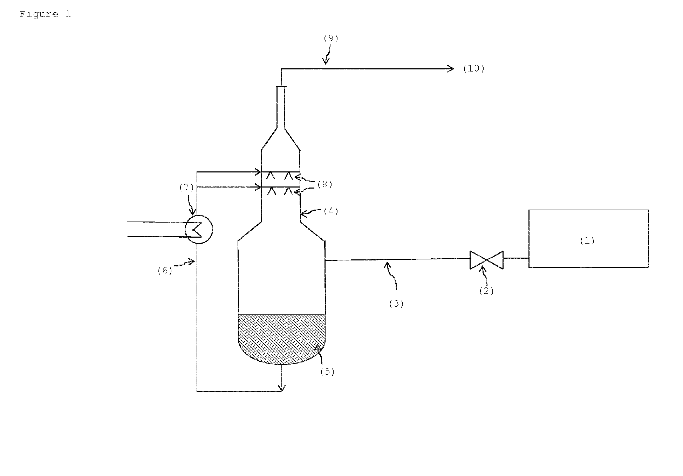

Figure 1 shows an example of apparatus according to the invention.

### Detailed Description of the Invention

In a preferred embodiment of the invention, the chemical plant is a plant for preparing diaryl carbonate. Preferably the diaryl carbonate is prepared from a dialkyl carbonate and an aryl alcohol. Most preferably dialkyl carbonate is converted into a diaryl carbonate via two steps. In a first step transesterification of the dialkyl carbonate with the aryl alcohol takes place to yield alkyl aryl carbonate and alkyl alcohol. In a second step disproportionation of the alkyl aryl carbonate takes place to yield diaryl carbonate and dialkyl carbonate. Further transesterification of the alkyl aryl carbonate with aryl alcohol may also take place to yield diaryl carbonate and alkyl alcohol.

In a most preferred embodiment of the invention, the chemical plant is a plant for preparing diphenyl carbonate. Preferably the diphenyl carbonate is prepared from diethyl carbonate and phenol. Reaction preferably takes place in the presence of a transesterification catalyst. Suitable catalysts and reaction conditions are known to the skilled person and are described, for example, in US5344954. In a diphenyl carbonate plant there will be vessels containing phenol, diethyl carbonate and diphenyl carbonate and all these compounds have low melting points and therefore could potentially solidify in relief, vent or flare lines. Using the apparatus or process of the invention ensures that, in the event of unwanted pressure increase, gases may be released via the relief line to the scrubber wherein the solvent is capable of dissolving compounds such as phenol, diethyl carbonate and diphenyl carbonate. By removing these low melting point compounds in the scrubber, the risk of having compounds solidify and block lines in the plant is mitigated.

In an alternative embodiment of the invention, the chemical plant is a plant for preparing a mixed alkyl aryl carbonate. Preferably the mixed alkyl aryl carbonate is prepared from a dialkyl carbonate and an aryl alcohol.

In another alternative embodiment of the invention, the chemical plant is a plant for preparing a polycarbonate. In a polycarbonate plant, a diaryl carbonate such as diphenyl carbonate is polymerised with a dihydroxy aromatic compound such as bisphenol A (4,4'-(propan-2-ylidene)diphenol), thereby producing the polycarbonate and phenol. In such a plant there will be vessels containing phenol, diphenyl carbonate and bisphenol A and all these compounds have low melting points and therefore could potentially solidify in relief, vent or flare lines. Using the apparatus or process of the invention ensures that, in the event of unwanted pressure increase, gases may be released via the relief line to the scrubber wherein the solvent is capable of dissolving compounds such as phenol, diphenyl carbonate and bisphenol A. By removing these low melting point compounds in the scrubber, the risk of having compounds solidify and block lines in the plant is mitigated.

The vessel in the apparatus or process of the invention that has a relief valve leading to a relief line may be a storage tank, a distillation column, an accumulator vessel, a reactor or any other vessel in the plant wherein undesirably high pressure may need to be reduced. Examples of situations wherein pressure may build up in these vessels include overfilling a storage tank, overheating a distillation column, fire in an accumulator vessel or runaway reaction in a reactor. Suitably there will be multiple vessels in the plant that have relief valves and suitably these will all lead to relief lines that provide the gas stream from the vessel to one or more scrubbers, and a solvent is present in the one or more scrubbers and the solvent contacts the gas stream in the one or more scrubbers and the solvent is an organic solvent.

The relief valve opens when the pressure in the vessel goes above a threshold value thereby allowing a gas stream to enter the relief line. The threshold value is determined by the skilled person and will vary depending on the type of vessel and its operating conditions but is typically greater than the back pressure of any flare line.

The relief line is suitably designed to mitigate the risk of low melting point components solidifying within the relief line. One means of achieving this is to minimise the length of the relief line. A preferred method is to provide external heating to the relief line. Desirably the exterior of the relief line is kept at a temperature of at least 100°C. This can be accomplished by electrical heating or by using steam.

The scrubber is suitably a conventional scrubber such as a spray tower, a baffle spray scrubber or a venturi scrubber. Preferably the solvent takes up at least 10vol% of the volume of the scrubber, more preferably at least 20vol%, most preferably at least 25vol% of the scrubber. The solvent is typically positioned at the base of the scrubber but is suitably recirculated from the base of the scrubber to the side or top of the scrubber. Preferably the solvent is sprayed into the side or top of the scrubber.

In a preferred embodiment of the invention the solvent in the scrubber is cooled. This is typically accomplished by passing the solvent through a heat exchanger. In a preferred embodiment, the solvent is recirculated from the base of the scrubber to the side or top of the scrubber and the heat exchanger is positioned in the recirculation loop. In an alternative embodiment, the solvent may be cooled by a heat exchanger which is located within the scrubber, e.g. cooling coils could be positioned within the solvent in the scrubber. Cooling is important because, without cooling, the temperature of the solvent might continue to increase until the solvent started to evaporate and then the solvent would be lost. Cooling is also important because at temperatures above around 250°C, components such as diethyl carbonate and diphenyl carbonate may undergo decomposition, which can lead to further increase in pressure.

The size of the scrubber and the rate of recirculation is suitably chosen by the skilled person bearing in mind the maximum relief scenario, i.e. the situation where the scrubber would be subjected to the greatest volume of gas via the relief line. The skilled person will also consider the amount of cooling of the solvent that may be required and the length of time during which the scrubber may have to operate during an emergency situation. Suitably the scrubber has a volume of at least 5m³, preferably at least 8m³. The scrubber is preferably located separately from the fire zone of the chemical plant. Preferably the scrubber has its own bund and power emergency system.

A gas stream leaves the scrubber and may be supplied to a flare or may be vented. The scrubber suitably does not have a relief valve and preferably has an open connection to flare lines and a flare. The gases leaving the scrubber will not contain components such as phenol, diethyl carbonate and diphenyl carbonate as these will have been absorbed by the solvent in the scrubber. Therefore in a preferred embodiment the apparatus of the invention further comprises a flare line, wherein the flare line provides gases from the scrubber to a flare, and wherein the flare line is not heated. It is not necessary to heat any flare lines as the present invention advantageously removes low melting point compounds in the scrubber, and reduces the risk of having compounds solidify and block the flare lines. Additionally, during an emergency situation it is often impossible to heat flare lines (e.g. because there is no electricity) and therefore it is desirable to be able to operate the apparatus without having to heat the flare lines.

The solvent in the scrubber will gradually change composition as it removes components from the gas stream and as small amounts of solvent evaporate. Suitably the skilled person will periodically add additional solvent to ensure that sufficient solvent is present in the scrubber. After an event wherein pressure in a vessel was reduced by supplying a gas stream via the relief line to the scrubber, the skilled person will suitably take samples of the solvent from the scrubber and then determine whether it is necessary to replace the solvent, e.g. because of the presence of too much phenol or diphenyl carbonate in the solvent. The solvent that is removed from the scrubber can be treated as waste or can be reprocessed to capture valuable components that may be present.

The solvent is an organic solvent. Possible solvents include non-polar solvents such as toluene; polar protic solvents such as methanol, ethanol, isopropyl alcohol and 2-ethyl phenol; and polar aprotic solvents such as organic carbonates, ethyl acetate, acetone and dimethylformamide. In a preferred embodiment, the solvent is a polar aprotic solvent. Suitably the solvent is chosen from the group consisting of dialkyl carbonates, alkyl aryl carbonates and diaryl carbonates, wherein the alkyl groups are preferably C₁₋₄ groups and the aryl groups are preferably phenyl groups. Preferably the solvent is a dialkyl carbonate, wherein the alkyl groups are C₁₋₄ groups. More preferably the solvent is diethyl carbonate or dimethyl carbonate. Most preferably the solvent is diethyl carbonate. When the chemical plant is a plant for preparing diphenyl carbonate from diethyl carbonate and phenol it is particularly preferred to use diethyl carbonate as the solvent because diethyl carbonate is readily available as it is used as a feedstock in the plant.

It is possible that the solvent could be a mixture of two or more different solvents, e.g. a commercial mixture of different dialkyl carbonates. It is not essential that the solvent is a high-grade solvent of high purity; less pure grades can also be used. For example, the solvent could contain up to 20wt% impurities based upon the weight of the solvent. These impurities could include pre-reactants used to make the solvent, e.g. alcohols that are used to make dialkyl carbonates.

Figure 1 shows an example of apparatus according to the invention for use in a chemical plant wherein diethyl carbonate and phenol are reacted to provide diphenyl carbonate. A vessel (1), which could be a storage tank, a distillation column, an accumulator vessel or a reactor, has a relief valve (2) which leads to a heated relief line (3). During an emergency event, e.g. overfilling of a storage tank, there will be an increase in pressure in the vessel (1). Once the pressure reaches a threshold value, the relief valve (2) will open and a gas stream from the vessel (1) will enter the relief line (3). The relief line (3) provides the gas stream from the vessel (1) to the scrubber (4). The scrubber (4) contains a solvent (5) which is diethyl carbonate. The solvent (5) is recirculated from the bottom of the scrubber (4) to the top of the scrubber (4) via a recirculation loop (6). There is a heat exchanger (7) in the recirculation loop (6) which cools the solvent. The solvent is reintroduced to the scrubber (4) via a series of nozzles (8) which spray the solvent into the scrubber (4). Low-boiling point components such as phenol, diethyl carbonate and diphenyl carbonate that are present in the gas stream originating from the vessel (1) are dissolved by the solvent. The gases leaving the scrubber (4) via a flare line (9) do not contain the low-boiling point components. The flare line (9) is unheated but is still unlikely to become blocked because the low-boiling point components have been removed by the scrubber (4).

The gases from the flare line (9) are directed to the flare (10).

## Claims

1. An apparatus for relieving pressure in a chemical plant, comprising:
a vessel that has a relief valve leading to a relief line, wherein the relief valve opens when the pressure in the vessel goes above a threshold value thereby allowing a gas stream from the vessel to enter the relief line;
and
a scrubber, wherein the relief line provides the gas stream from the vessel to the scrubber, wherein a solvent is present in the scrubber and the solvent contacts the gas stream in the scrubber and wherein the solvent is an organic solvent.

2. An apparatus according to claim 1, wherein the chemical plant is a plant for preparing diaryl carbonate, a plant for preparing a mixed alkyl aryl carbonate or a plant for preparing a polycarbonate.

3. An apparatus according to claim 2, wherein the chemical plant is a plant for preparing diphenyl carbonate.

4. An apparatus according to any preceding claim, comprising a heat exchanger, such that the solvent in the scrubber is cooled by passing the solvent through the heat exchanger.

5. An apparatus according to any preceding claim, wherein the solvent is chosen from the group consisting of dialkyl carbonates, alkyl aryl carbonates and diaryl carbonates, wherein the alkyl groups are C₁₋₄ groups and the aryl groups are phenyl groups.

6. An apparatus according to claim 5, wherein the solvent is diethyl carbonate or dimethyl carbonate.

7. An apparatus according to any preceding claim, further comprising a flare line, wherein the flare line provides gases from the scrubber to a flare, and wherein the flare line is not heated.

8. A process for relieving pressure in a chemical plant, comprising steps of:
opening a relief valve between a vessel and a relief line when the pressure in the vessel goes above a threshold value, thereby allowing a gas stream to enter the relief line;
supplying the gas stream from the relief line to a scrubber;
contacting the gas stream with a solvent in the scrubber;
wherein the solvent is an organic solvent.

9. A process according to claim 8, wherein the chemical plant is a plant for preparing diaryl carbonate, a plant for preparing a mixed alkyl aryl carbonate or a plant for preparing a polycarbonate.

10. A process according to claim 9, wherein the chemical plant is a plant for preparing diphenyl carbonate.

11. A process according to any one of claims 8 to 10, comprising a further step of passing the solvent through a heat exchanger and thereby cooling the solvent.

12. A process according to any one of claims 8 to 11, wherein the solvent is chosen from the group consisting of dialkyl carbonates, alkyl aryl carbonates and diaryl carbonates, wherein the alkyl groups are C₁₋₄ groups and the aryl groups are phenyl groups.

13. A process according to claim 12, wherein the solvent is diethyl carbonate or dimethyl carbonate.

14. A process according to any one of claims 8 to 13, comprising a further step of providing gases from the scrubber to a flare via a flare line, wherein the flare line is not heated.
